# EUROPEAN PATENT SPECIFICATION

(11) **EP 0 514 051 B1**
(45) Date of publication and mention of the grant of the patent: **21.06.1995**
(21) Application number: 92303961.4
(22) Date of filing: 30.04.1992
(51) Int. Cl.: A61B 17/22

(54) **Ultrasonic therapy apparatus**
Ultraschalltherapievorrichtung
Dispositif de thérapie par ultrasons

(30) Priority: 01.05.1991 JP 100160/91; 15.11.1991 JP 300292/91
(43) Date of publication of application: 19.11.1992
(73) Proprietor: KABUSHIKI KAISHA TOSHIBA, Kawasaki-shi, Kanagawa-ken 210 (JP)
(72) Inventor: Iwama, Nobuyuki, c/o K. K. Toshiba, Intellectual, Minato-ku, Tokyo 105 (JP); Okazaki, Kiyoshi, c/o K. K. Toshiba, Intellectual, Minato-ku, Tokyo 105 (JP); Kanaya, Yasuhiro, c/o K. K. Toshiba, Intellectual, Minato-ku, Tokyo 105 (JP)
(74) Representative: BATCHELLOR, KIRK & CO.

(56) References cited:
- EP-A- 0 169 311
- EP-A- 0 260 550
- EP-A- 0 405 282
- DE-A- 3 429 882
- FR-A- 2 488 094

## Description

The present invention relates to an ultrasonic therapy apparatus for destroying an object, e.g., cancer cell, stone, in a patient by means of a focused energy of shock wave, or for performing other such treatment.

A conventional ultrasonic therapy apparatus, for example, extracorporeal shock wave treatment apparatus comprises an applicator, applicator support unit, a couch for supporting a patient, shock wave control device, tomographic imaging display unit, and water control devices etc. The applicator includes a shock wave transducer which has a plurality of electro-acoustic transducer elements for transmitting a focused shock wave to a predetermined direction and depth of the patient. The transmission side of the applicator is directed downwardly towards the patient, and transmitted shock wave from the transducer can then be used to destroy a kidney stone or gallstone in the patient.

Thus in a conventional shock wave treatment apparatus, the applicator is arranged above the patient and transmits the shock wave to the patient downwardly. This arrangement of applicator is referred to as the downward approach type. The alternative arrangement, in which the applicator is arranged below the patient, and transmits the shock wave upwardly to the patient, is referred to as the upward approach type. An advantage of the downward approach type is its ease of operation in focussing the shock wave onto a calculus. Thus, the operator can adjust the position of the applicator by co-ordinating several parameters, e.g. the position and body surface of the patient, the position of the applicator, and the contact condition between the patients and the applicator.

Although upward approach type has the merit of easy operation for adjusting the focus position with respect to the calculus, it also has disadvantages when the therapy object is a gallstone.

When the gallstone is destroyed by a shock wave from an applicator of upward approach type, the patient must lie prone. In the prone position, the gallstone is positioned beneath a rib and a lung. Hence transmitted shock wave is absorbed and attenuated by air in the lung. Also, in shock wave therapy apparatus using a ultrasonic imaging probe positioned centrally of the applicator, structure below the lung is not displayed on the ultrasonic tomographic image, because the ultrasonic beam from the imaging probe is also absorbed and attenuated by air in the lung. Further, the gallstone is moved by patient movement caused by the breathing, for example, hence the above operation for adjusting the focus position onto the gallstone is very difficult.

When an applicator using the downward approach is contacted with the patient, the distance between the applicator and the kidney stone in the patient is approximately 5cm to 12cm, and when the applicator using the upward approach is contacted with the patient, the distance between the applicator and the gallstone in the patient is approximately 2cm to 7cm. As is well known in an ultrasonic technology, the attenuation coefficient of tissue is related to the depth of object. The attenuation coefficient of tissue increases substantially linearly with frequency, with the high frequency spectral components of a returned signal being attenuated more severely than the low frequency components. Typically, the centre frequency of the received signal drops in frequency with depth of penetration.

Such problems relate to the shock wave treatment apparatus, and also to therapy involving hyperthermia with the downward approach.

Accordingly the present invention seeks to provide an improved ultrasonic therapy system for focussing ultrasound onto an object to be destroyed.

The reader will find further guidance as to the state of the art in EP-A-405 282, with reference to which claim 1 of this application is characterised.

According to the present invention there is provided an ultrasonic treatment apparatus as defined in claim 1.

The ultrasonic therapy apparatus of the present invention can thus be used to select the downward approach or upward approach, and the approach direction can be clearly indicated by the displayed tomographic image. Accordingly, for example, the downward approach is selected when the object to be treated is a kidney stone, and the upward approach is selected when the object to be destroyed is a gallstone, and these approach directions can be confirmed by displayed tomographic image.
Figure 1 is a perspective view of a system of the ultrasonic therapy apparatus set in a downward approach condition according to the present invention;
Figure 2 is a perspective view of a system of the ultrasonic therapy apparatus set in an upward approach condition according to the present invention;
Figure 3 is a block diagram showing an arrangement of a shock wave treatment apparatus of the present invention;
Figure 4 shows a control panel of the apparatus of Figure 2;
Figure 5 illustrates how the downward approach condition is indicated on a monitor;
Figure 6 illustrates how the upward approach condition is indicated on a monitor;
Figure 7 is a modified form of the ultrasonic imaging system shown in Figure 3;
Figure 8 is a top view, with portions broken away for clarity, of an applicator and an applicator support device shown in Figure 3;
Figure 9 is a front view of the apparatus of Figure 8;
Figure 10 is a side view of the apparatus of Figure 8;
Figure 11 to 13 are illustrative diagrams showing an example of the applicator inclination mechanism of Figure 8;
Figures 14 and 15 illustrate different relative positions of the applicator and the patient;
Figure 16 is a modified embodiment of the apparatus of Figure 10;
Figure 17 is a partial sectional view of the device shown in Figure 16;
Figure 18 is a second embodiment showing a condition of the upward approach displayed on a monitor shown in Figure 3;
Figure 19 is a second embodiment of Figure 17;
Figure 20 is a preferred embodiment of a couch for a patient shown in Figure 1; and
Figure 21 is a second embodiment of the applicator support device shown in Figure 1.

An embodiment of a shock wave treatment apparatus according to the present invention will now be described with reference to the accompanying drawings.

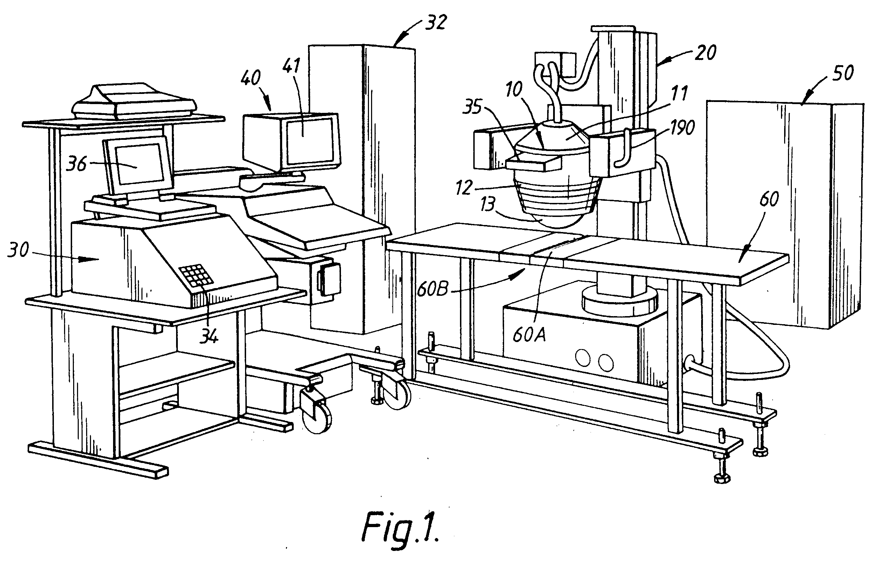

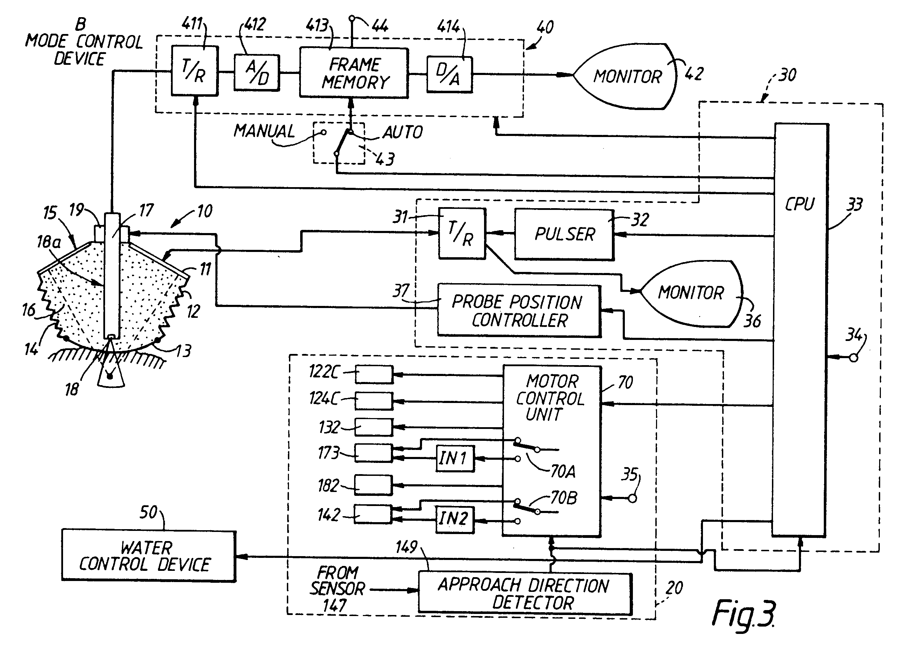

Referring now to Figures 1 to 3, the shock wave treatment apparatus of the first embodiment consists of an applicator 10, applicator support device 20, shock wave control device 30, ultrasonic tomogram imaging device 40, water control device 50, and couch 60. The applicator 10 (Figure 3) comprises a shock wave generator 11 which has a through hole at a centre portion, a bellows 12, diaphragm 3, and the applicator 10 forms an airtight container 14. The airtight container 14 is fixed to a frame 15, and is filled with water 16 as an ultrasound penetration material. A rod member 17 is inserted into the airtight construction 14 through the through hole of the frame 15 and the shock wave generator 11. An ultrasonic transducer 18 for observing the tomographic image is arranged in an end of rod member 17, hence an ultrasonic probe 18a constructed therefrom. The frame 15 has a probe moving mechanism 19 which allows to move the ultrasonic probe 18a within the airtight construction 14 toward a downward and upward direction. The shock wave generator 11 is formed by a plurality of high power type ultrasonic transducers which are arranged in a circle on the frame 15 around the through hole of the frame 15. In this arrangement of ultrasonic transducer, transmitted shock wave form a focus point at a predetermined depth which depends the radius of the concave shape of shock wave generator 11.

The applicator support device 20 supports the applicator 20 which permits some movement, i.e. horizontal (XY-axis), large up and down (large Z-axis), small up and down (small z-axis), turn over, and inclination movement. The applicator support device 20 will be described in more detail below.

Referring now to Figure 3, the shock wave control device 30 consists of a ultrasonic transmitting and receiving unit (T/R) 31, a pulser 32, a central processing unit (CPU) 33, a fixed operation panel 34, an operation panel 35 which can be easy put on and take off, a monitor 36, and a probe position controller 37. The T/R 31 permit to supply high power or low power pulse from the pulser 32 to the shock wave generator 11 selectively. Here, the high power pulse is generated for obtaining the shock wave, and the low power pulse is generated for confirming a condition of coincidence between the focus of shock wave and the calculus. A statistics data obtained as a number of emit etc. of the high power and the low power pulses is displayed on the monitor 36. The probe position controller 37 controls the downward/upward movement of the ultrasonic probe 18a by control signal from the CPU 33, and sets the ultrasonic probe 18a to predetermined position within the airtight construction 14. The operation panel 35 which can be easy put on and took off is fitted to the frame 15 of the applicator 10. This operation panel 35 has five operation switches for the movement of the applicator, i.e. first one 35a for the inclination movement along the turn over direction, second one 35b for the inclination movement along in front and behind direction of the applicator 10, third one 35c for XY movement along the XY-axis, fourth one for the small up and down movement along the small z-axis, and fifth one 35e for the large up and down movement along the large Z-axis.

The ultrasonic tomogram imaging device 40 comprises a B-mode control device 41 and a monitor 42. More particularly, the B-mode control device 41 consists of a transmission and reception unit (T/R) 411, an A/D converter 412 for converting supplied analog data to digital data, a frame memory 413 for storing the digital data from the A/D 412, a D/A converter 414 for converting the digital data supplied from the frame memory to analog data and, the analog data from the D/A 414 is supplied to the monitor 42, hence a ultrasonic tomographic image is displayed on the monitor 42. Here, an upper and lower of stored tomogram data in the frame memory 413 is turned over based on a control signal from CPU 33 via a switching unit 43 or a turnover switch 44 which permit to direct the turn over in desired timing, and the switching unit 43 is changed between an AUTO position and a MANUAL position according to operate the switch by operator.

The applicator support device 20 includes a number of motors, and circuitry for controlling the motors. As shown in Figure 3, an output of a motor control unit 70 which has two switches 70a and 70b is connected to motor 122c, 124c, 132 and 182 directly, and is connected to motor 173, and 142, both directly and via inverter circuits IN 1 and IN 2 respectively. The switches 70a and 70b can select a direct connection, or inverted connection via the inverter circuits IN 1 and IN 2, with the motor 173 and 142. The switches 70a and 70b are selected any terminal based on an output of an approach direction detector 149 which will be described below. The function of the motors are as follows: the motor 122c displaces the applicator support device 20 along the X-axis direction, the motor 124c displaces the applicator support device 20 along the Y-axis direction, the motor 132 moves the applicator 10 along the Z-axis direction (large movements), the motor 173 moves the applicator 10 along the z-axis direction (small movements), the motor 182 inclines the applicator 10, and the motor 142 rotates the applicator 10.

The water control device 50 controls supplying and draining the water 16 within the airtight construction 14 of the applicator 10. According to this water control, a depth of the shock wave focus within the patient is adjusted to a suitable depth for treating the calculus. A table top 60a to carry the patient has an opening and shutting aperture 60b. The aperture 60b is opened when the upward approach for shock wave treatment is selected by operator.

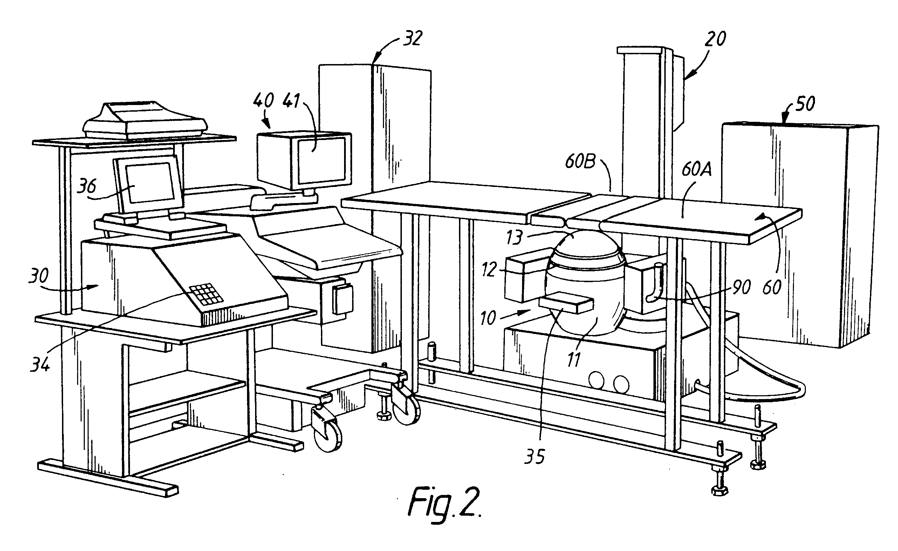

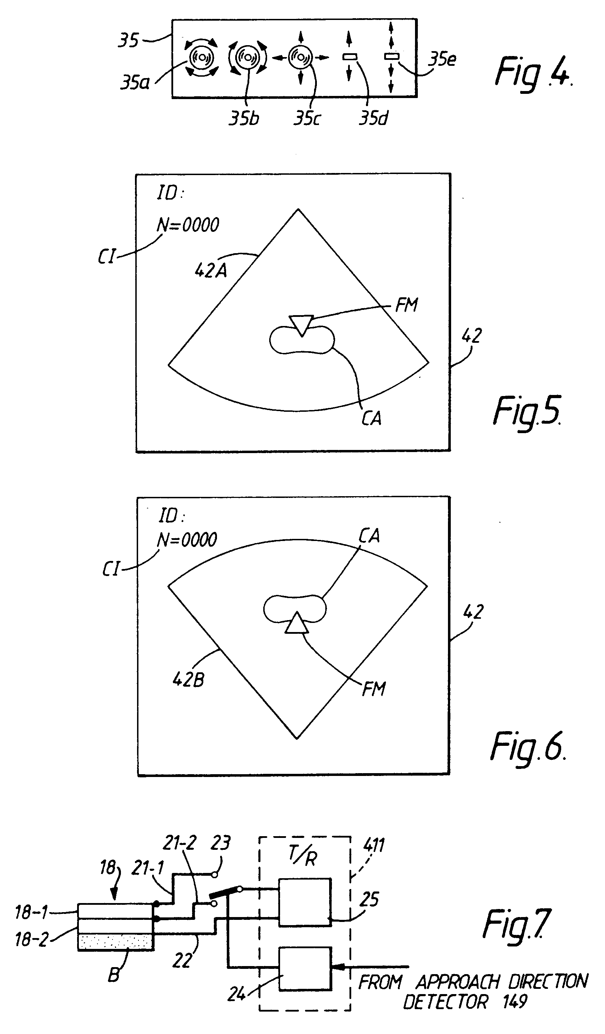

In the above construction, the applicator 10 can be set a position for downward approach as shown in Figure 1, and the applicator 10 can alternatively be set to a position for upward approach as shown in Figure 2. This selection of the approach direction is allowed by operation of the applicator support device 20. If the applicator 10 is set for the downward approach, a sector tomographic image 42a is displayed on the monitor 42 with focus marker FM as shown in Figure 5, and if the applicator 10 is set for the upward approach, the sector tomographic image 42b is displayed in upside down on the monitor 42 with focus marker FM as shown in Figure 6. Consequently, the approach condition, downward or upward, the focus position of shock wave, and the position of calculus can be observed easily. A "CI" field in the image shows character information relating to patient and/or shot No. of shock wave etc., and a "CA" field shows the calculus image.

Further, the ultrasonic transducer may be modified as shown in Figure 7, namely, ultrasonic transducer 18 consists of two transducers 18-1 and 18-2 on a backing member B. The transducer 18-1 is sandwiched by two electrodes 21-1 and 21-2 for energising the transducers, and the transducer 18-2 is sandwiched by the electrode 21-2 and an earth 22 which is on the backing member B. The electrodes 21-1 and 21-2 to be connected with pulser 25 is selected by switch 23. The switch 23 is changed by a selector 24 which is controlled by the output of the approach direction detector 149. As a result of the above transducer construction, frequency of the emitted ultrasound may change, namely, frequency of the emitted ultrasound is certified depend on a thickness of transducer, hence the switch 23 selects the appropriate one. When both the transducers 18-1 and 18-2 are energised, then the frequency of the emitted ultrasound becomes 3.5 MHz, and when only the transducer 18-2 is energised, then the frequency of the emitted ultrasound becomes 5 MHz. The ultrasound frequency of 3.5 MHz is used when the treatment object is the kidney stone, the ultrasound frequency of 5 MHz is used when the treatment object is the gallstone.

When the applicator using the downward approach is contacted with the patient, the kidney stone is located in a relatively deep position. Then the ultrasound frequency is used 3.5 MHz, hence the kidney stone located to the deeper position can be observed clearly. When the applicator using the upward approach is contacted with the patient, gallstone located to relatively slight position. Then the ultrasound frequency is used 5 MHz, hence the gallstone located to relatively slight position also can be observed with high contrast image.

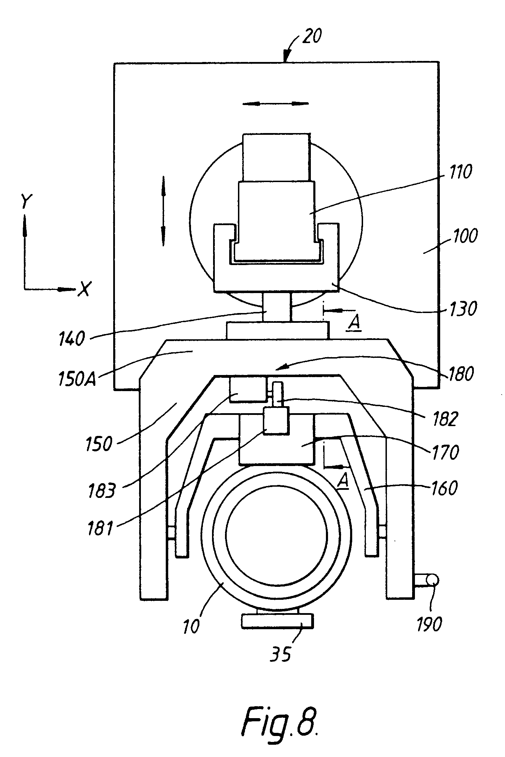

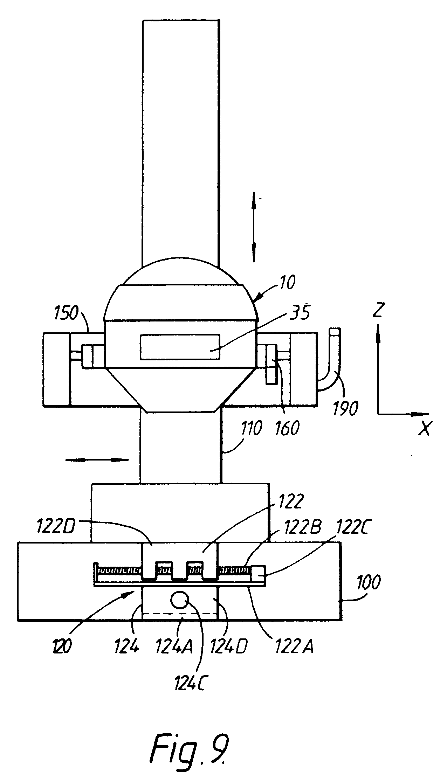

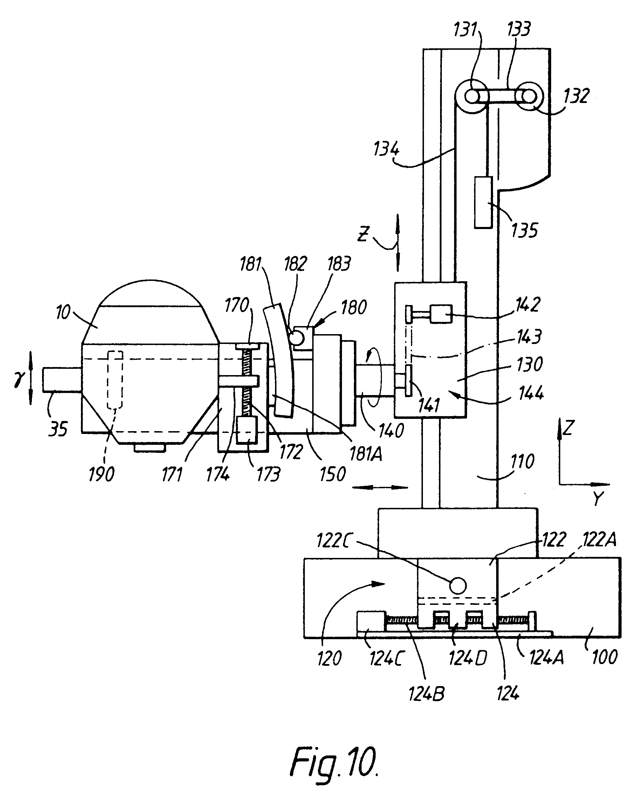

A detailed embodiment of the applicator support device 20 will now be described. As shown in Figure 8, a base 100 set on the floor supports a tower 110. This tower 110, as shown in Figure 9 and Figure 10, is permitted to displace toward both X-axis and Y-axis direction by XY-axis displacement device 120 provided within the base 100. This XY-axis displacement device 120 comprises a Y-axis displacement mechanism 124 mounted on an X-axis displacement mechanism 122 a shown in Figure 9. The displacement mechanisms both incorporate leadscrews. As shown in Figure 9, the X-axis displacement device 122 comprises a rotatable screw 122b provided on a X-axis fixed plate 122a. The screw 122b can be rotated by means of a motor 122c positioned on one end of the screw. An X-axis lead 122d is engaged with the screw 122b, hence the X-axis lead 122d can be moved along the X-axis direction by driving the motor 122c. As shown in Figure 10, the Y-axis displacement device 124 comprises a rotatable screw 124b provided on a Y-axis fixed plate 124a. The screw 124b can be rotated by rotating a motor 124c positioned on one end of the screw. A Y-axis lead 124d is engaged with the screw 124b, hence the Y-axis lead 124d can be moved along the Y-axis direction by driving the motor 124c. The Y-axis fixed plate 124a is fixed to the base 100, and the X-axis fixed plate 122a is coupled to the tower 110. Consequently, the tower 110 can be displaced toward both the X-axis and Y-axis direction individually by means of driving the motor 122C and 124C of the XY-axis displacement device 120.

In Fig.10, the tower 110 has a C-shaped sliding member 130, and the sliding member 130 can be slided along a Z-axis on the tower 110. This tower 110 also has a sprocket 131 and a motor 132 in an end of the tower 110, and the sprocket 131 and the motor 132 are engaged by a chain 133. The sprocket 131 is also engaged by a wire 134 which connect to the sliding member 130 at an one end 134 and connect to a balance weight 135 at an other end. Hence, when the motor 132 is energized, sliding member 130 is adjusted to desired position along the Z-axis on the tower 110. In other ward, the applicator 10 permit to move along the Z-axis direction.

The sliding member 130 has a rotating axis 140 which has a sprocket 141 at an one end of the rotating axis 140. An other end of the rotating axis 140 is fixed to a C-shaped arm 150. And the sliding member 130 has a arm rotation mechanism 144 which comprises a sprocket 141, motor 142, and chain 143 engaged between the sprocket 141 and motor 142. Hence, by means of energizing the motor 142, the C-shaped arm 150 permit to rotate around an arrow r as shown in Fig.10. In other word, the direction of emitting the shock wave from the applicator 10 permit to change between the downward approach and the upward approach at desired timing.

The C-shaped arm 150 has a C-shaped support 160 which connect to the C-shaped arm 150 at axis 161 provided on both end of the C-shaped support 160. A mid portion of the C-shaped support 160 is fixed an applicator support base 170 which supports the applicator 10 slidably to the C-shaped support 160. The applicator support base 170 has a screw mechanism 171 which includes a screw 172, a motor 173 for rotating the screw 172, and a lead 174 engaged with the screw 172. And an end portion of the lead 174 is fixed the applicator 10. Consequently, applicator 10 can be moved along the z-axis by rotating the motor 173.

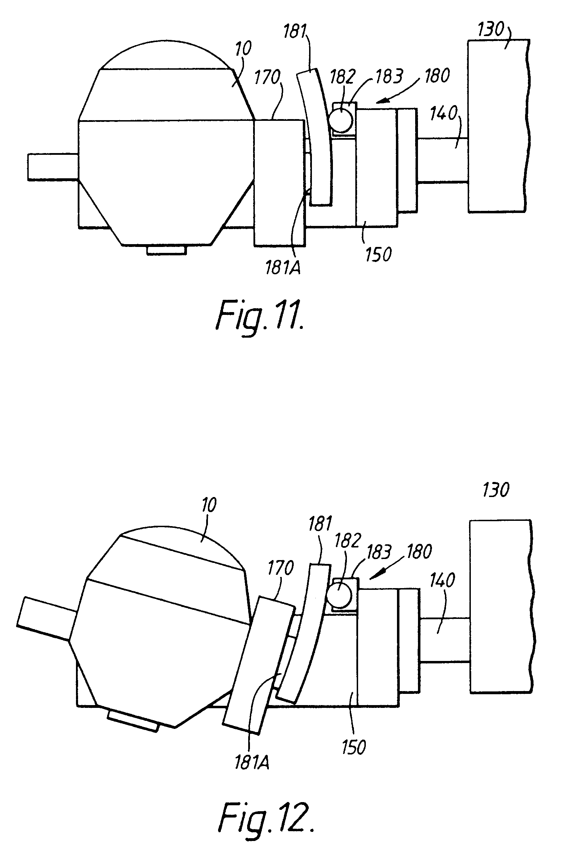

A base portion of the C-shaped arm 150 has an inclination mechanism 180. This inclination mechanism 180 consists of a curved rack gear 181 fixed to the applicator support base 170 by an attachment 181A, a pinion gear 182 for engaging with the curved rack gear 181, a motor 183 for rotating the pinion gear 182. In the above construction, when the motor 183 is energized based on the control signal from the second switch 35b, then the curved rack gear 181 is slid toward the up or down direction. Consequently, the applicator 10 integrated with the C-shaped support 160 is inclined as shown in Fig.11 to Fig.13.

A grip 190 is fixed to the arm portion of the C-shaped arm 150. By means of the grip 190 the applicator 10 permits setting the best suited position for treatment operation and also turning over between the downward approach and the upward approach by hand-powered operation.

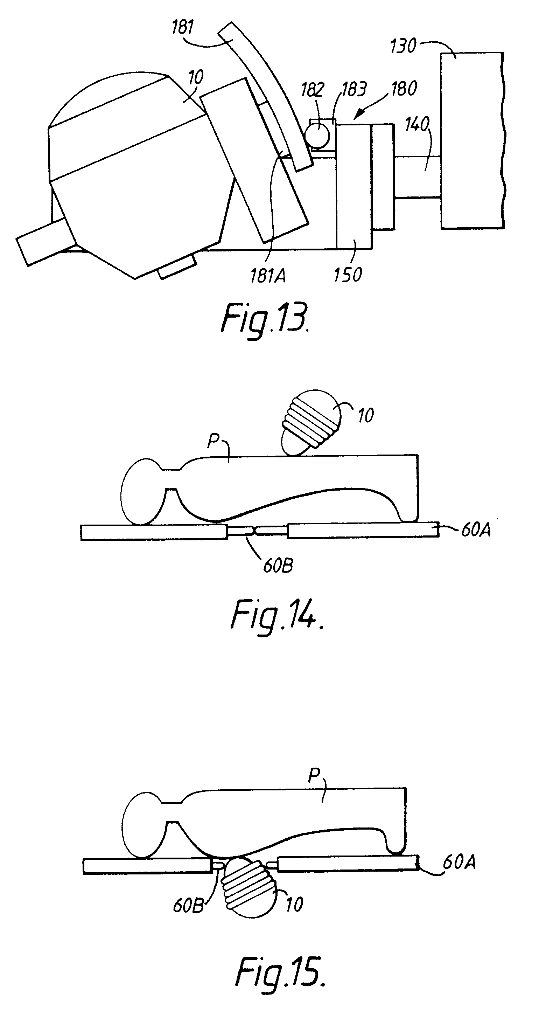

As described above, according to the present embodiment, the operator can selects the one from between the downward approach and the upward approach, moreover the operator easily permit to operate for coinciding the focus point of the shock wave to the calculus in both the downward and upward approach. Hence, as shown in Fig.14 and Fig.15, this shock wave treatment apparatus is very convenient for treatment operation of both the gallstone and the kidney stone. In this case, when the downward approach is selected, then the aperture portion 60B is closed, and when the upward approach is selected, then the aperture 60B is opened. Then, the selection of which between the downward and the upward approach is achieved by controlling the arm rotation mechanism 144 within the sliding member 130. The operator can supply a command to the CPU 33 for moving the applicator toward the several direction, i.e. horizontal (XY-axis), large up and down (large Z-axis), small up and down (small z-axis), turn over, and inclination movement, then, the command from the CPU supply to the motor corresponded to the command provided to the fixed operation panel 34 or the operation panel 35 which can be easy put on and take off, by the operator. The operation panel 35 can be removed once when the applicator 10 is turned over from the downward to upward approach. After that, the operation panel 35 is fitted on the applicator 10 again. In here, when the approach direction of the applicator 10 is changed, then the movement direction instructed by the operation panel 35 regarding small z-axis and rotation movement is reversed against an indicated arrow on the operation panel 35. However, in this invention, this reversed movement regarding small z-axis and rotation movement can be prevented by an effect of the inverter circuit IN 1 and IN 2 shown in Fig.2. Namely, when the approach direction is changed from the downward to upward, the switch 70A and 70B within the motor control unit 70 is switched to side of the inverter circuit IN 1 and IN 2 respectively based on the output signal of the approach direction detector 149. According to the above switching, the control signal from the switches 35a and 35d within the operation panel 35 is inverted by the inverter circuit N 1 and IN 2 respectively. Hence although the approach direction of the applicator is turned over, the operator can operates along the arrow indicated on the operation panel 35.

Moreover, the operation panel 35 allows to operate the applicator for positioning in remote control from detached area.

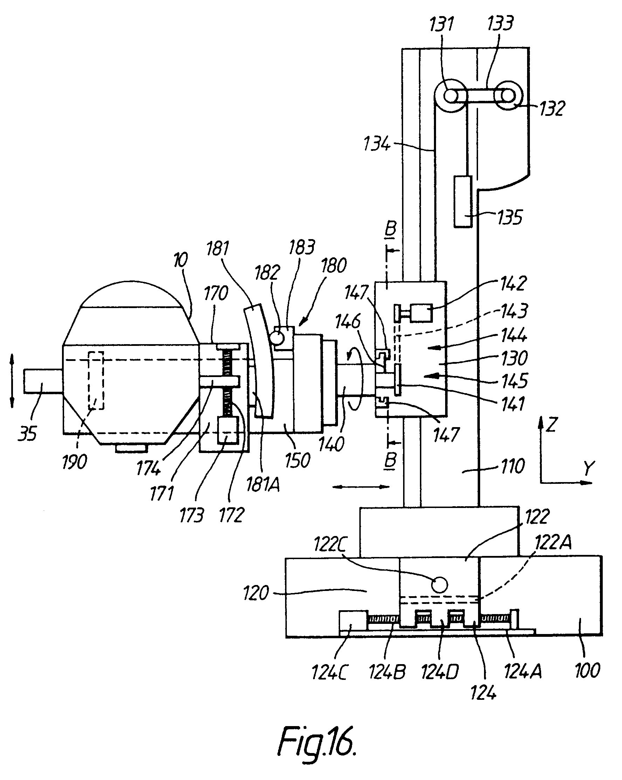

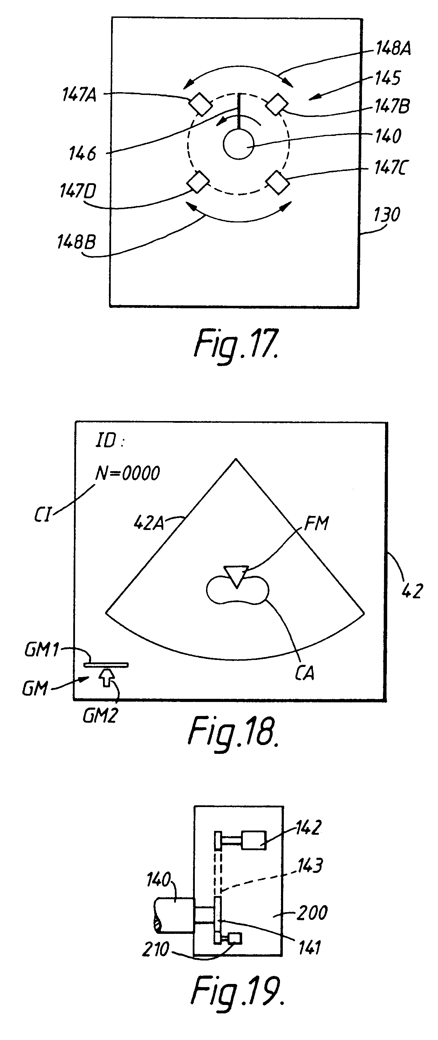

Second embodiment will be described below Referring to Fig.16 and Fig.17. The gist of the second embodiment has a function that displayed B-mode image on the monitor 42 is turned over automatically based on the approach condition as the downward or the upward approach. Namely, as shown in Fig.16, rotation position detecting device 145 is provided within the sliding member 130. This rotation position detecting device 145 comprises a rod 146 fixed to the rotating axis 140 and four sensors 147A, 147B, 147C, and 147D for detecting a position of the rod 146. The sensors 147A to 147D are arranged along a locus of the rotated rod 146 at a equal interval each other for detecting the position of the rod 146 rotated integrally with the rotating axis 140. An output of the sensors 147A to 147D are supplied to a approach direction detector 149 shown in Fig.3. This approach direction detector 149 judges whether the applicator 10 is in the downward approach or is in the upward approach based on the output of the sensors 147A to 147D indicated the position of the rod 146. Especially, as shown in the Fig.17, when the rod 146 is in an area 148A lied between the sensor 147A and 147B, then the approach direction detector 149 judges to downward approach condition, and the rod 146 is in an area 148B lied between the sensor 147C and 147D, then the approach direction detector 149 judges to upward approach condition.

A judgement signal of the approach condition obtained the approach direction detector 149 is supplied to the CPU 33 via the applicator 20. The CPU 33 supplies a control signal to the B-mode control device 41 via the switching unit 43 for setting the displayed condition of the B-mode image displayed on the monitor 42 based on the judgement signal of the approach condition.

This B-mode control device 41 is connected with a switch unit 43 which can be changed manually between the AUTO position and the MANUAL position. When the AUTO position is selected, then the displayed condition is changed by the control signal regarding approach condition from the CPU 33. For example, when the applicator is in the downward condition, the sector tomographic image is displayed on the monitor as shown in Fig.4, and when the applicator is in the upward condition, the sector tomographic image is displayed in upside down on the monitor 42 as shown in Fig.5. On the other hand, when manual contact of manual is selected, then the displayed condition is changed based on a instruct signal from a turn over setting switch 44. As another way of the indication of the approach condition, graphical marker GM also can be displayed in side corner position of the monitor 42 as shown in Fig.18. Here, a bar mark GM1 shows the couch, and a arrow mark GM2 shows the approach direction of the applicator 10. Hence,the graphical marker GM shows the upward approach. When the MANUAL position is selected, there is no relation between the approach condition and the display condition on the monitor 42. In this state, the display condition on the monitor 42 is only changed based on the instruct signal from a turnover setting switch 44.

The above embodiment relates to the shock wave treatment apparatus, this invention also can be provided for the hyperthermia apparatus by changing from a shock wave generator to a continuous ultrasonic generator.

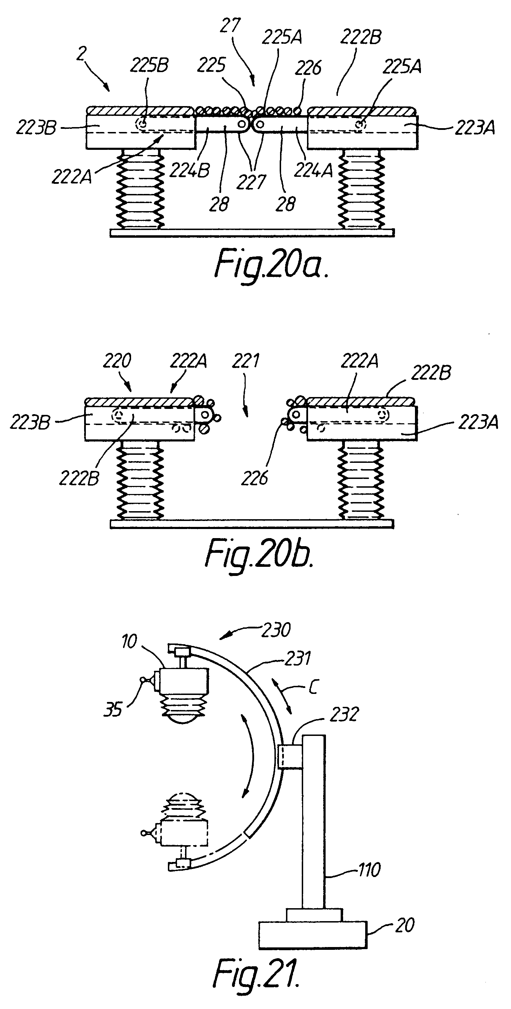

The present invention is not limited to the above embodiment, and various other changes and modifications may be made. For example, the rotation position detecting device 145 can be modified as 200 shown in Figure 19. The gist of this construction is that a potentiometer 210 is provided as substitution of the rod 146 and detectors 147A to 147D. Also the couch 60 can be modified as couch 220 shown in Figure 20 (a) and (b). Thus the aperture 221 has slide tables 222a and 222b which can be slid along the longitudinal of the couch 220, the slide tables 222a and 222b being housed in receiving spaces 223a and 223b. Further, the slide tables 222a and 222b are constructed with bases 224a and 224b which have rollers 225a and 225b provided at both ends of the base 224a and 224b, belt 226a and 226b hanging between both rollers 225a and 225b, and a plurality of cushion members 227 are attached to belt 226a and 226b, so that the slide table 222a and 222b can be slid in a horizontal direction without scraping the cushion members 227 against the patient. Furthermore, the applicator support device 20 can be modified as J-shaped support arm 230 which supports the applicator 10 at an end portion thereto, this J-shaped support arm 230 being supported by an axis 232 around the axis 232 and slidably along an arrow C. In this embodiment, when the operator desires to turn over the applicator, then the J-shaped support arm 230 can be rotated around the axis 232.

In the apparatus of this invention, the operator can select between the downward and the upward approach of the applicator 20 suitably, and this approach condition can be observed by the monitor which displayed the approach condition. Consequently, the operator selects the downward approach, when the position to be treated is the kidney stone, and the operator selects the upward approach, when the position to be treated is the gallstone, and these selected approach conditions can be observed on the monitor. The control signal from the switches 35a and 35d within the operation panel 35 is inverted by the inverter circuit N 1 and IN 2 respectively. Hence although the approach direction of the applicator is turned over, the movement direction is not inverted, so the operator can operate along the arrow indicated on the operation panel 35 without being concerned about the downward and upward approach of the applicator.

According to the embodiment described above, whatever approach direction is selected, the ultrasonic treatment apparatus of the present invention enable the operator to still easily control the positioning of the applicator.

## Claims

1. An ultrasonic treatment apparatus having an applicator (10) for generating and applying ultrasound to an object to be treated, and an applicator support means (20) for supporting the applicator (10) to be movable in different directions, said apparatus comprising:
an ultrasonic probe (18a) arranged in the applicator (10) for acquiring tomographic image information of the object to be examined and destroyed; and
means (144) for changing an approach direction of the applicator (10) between a downward approach and an upward approach relative to the object in order to achieve suitable treatment of the object to be treated;
monitor means (42) for displaying the tomographic image information of the object supplied from the ultrasonic probe (18A);
characterised in that said probe (18a) comprises a two layer transducer (18-1, and 18-2) on a backing member (B) for selectively emitting high and low frequency ultrasound, ultrasonic frequency conversion means (23 and 24) including a switch (23) provided for converting the ultrasound frequency emitted from the ultrasonic probe (18a) to the low frequency, when the upward approach is selected and being automatically switched by said switch (23) in response to the direction of approach of the ultrasonic probe (18a) detected by an approach direction detection means (145) which detects whether the approach direction is up or down.

2. An ultrasonic treatment apparatus according to claim 1 in which the approach direction is indicated on the monitor.

3. An ultrasonic treatment apparatus according to claim 2, wherein an approach direction indication means (40) indicates that the upward movement has been selected by producing the tomographic image upside down on the monitor.

4. An ultrasonic treatment apparatus according to claim 2, further comprising a graphical marker generating means for generating two graphical marks which respectively represent the table for supporting the object and an arrow corresponding to the direction of the applicator.

5. An ultrasonic treatment apparatus according to any preceding claim, wherein the applicator support means (20) comprises a base (100) fixed on a floor, an X-Y coordinate displacement means (120) mounted on the base (100) for displacing the applicator (10) on a two-dimensional plane, a tower (110) provided on the base (100), a Z direction displacement means (131, 132, 133, and 171) mounted on the tower (110) for displacing the applicator (10) perpendicular to the two-dimensional plane, an arm member (150) having a rotation axis (140) which is supported by the tower (110) along the rotation axis (140), and a rotation means (141, 142, 143 and 144) which is provided to rotate the arm member (150) for rotating the applicator (10) around the axis (140) horizontally arranged along the two-dimensional plane.

6. An ultrasonic treatment apparatus according to claim 5, wherein said approach direction detection means (145) includes four sensors (147a to 147d) connected to the tower (110) for detecting a rotation angle of said rotation axis (140) by detecting a part of the rotating structure lying between any two sensors.

7. An ultrasonic treatment apparatus according to claim 6, wherein said approach direction detection means includes a rotary potentiometer (210) for detecting the rotation angle.

8. An ultrasonic treatment apparatus according to any of claims 3 to 7, wherein said approach direction indicating means (40) can be controlled based on a turnover switch (44) which is operated manually by an operator.

9. An ultrasonic treatment apparatus according to claim 8, further comprising a switching unit (43) for selecting the indication of the approach condition whether automatically controlled based on the approach direction detection means (145) or manually controlled based on the turnover switch (44).

## Patentansprüche

1. Ultraschallbehandlungsvorrichtung, mit einem Applikator (10) zum Erzeugen und zum Anwenden von Ultraschall auf einen zu behandelnden Gegenstand und einer Applikatorhalteeinrichtung (20), um den Applikator (10) in verschiedene Richtungen bewegbar zu halten, wobei die Vorrichtung aufweist:
einen Ultraschallmeßfühler (18a), der im Applikator (10) angeordnet ist, um tomographische Bildinformationen des zu untersuchenden und zu zerstörenden Gegenstandes zu erhalten;
eine Einrichtung (144), um eine Bestrahlungsrichtung des Applikators (10) bezüglich des Gegenstandes zwischen einer nach unten gerichteten Bestrahlung und einer nach oben gerichteten Bestrahlung zu verändern, um eine geeignete Behandlung des zu behandelnden Gegenstandes zu erreichen; und
eine Monitoreinrichtung (42), um die vom Ultraschallmeßfühler (18a) gelieferten tomographischen Bildinformationen des Gegenstandes anzuzeigen;
**dadurch gekennzeichnet,** daß der Meßfühler (18a) einen auf einem Trägerbauteil (B) angeordneten Zweischicht-Meßwandler (18-1 und 18-2), um wahlweise Ultraschall mit hoher und geringer Frequenz zu emittieren, und eine Ultraschallfrequenz-Umschalteinrichtung (23 und 24) enthält, die mit einem Schalter (23) versehen ist, um die vom Ultraschallmeßfühler (18a) emittierte Ultraschallfrequenz auf die geringe Frequenz umzuschalten, wenn die nach oben gerichtete Bestrahlung gewählt ist, und die in Reaktion auf die Bestrahlungsrichtung des Ultraschallmeßfühlers (18a), welche durch eine Erfassungseinrichtung (145) für die Bestrahlungsrichtung erfaßt wird, die erfaßt, ob die Bestrahlung nach oben oder nach unten gerichtet ist, durch den Schalter (23) automatisch umgeschaltet wird.

2. Ultraschallbehandlungsvorrichtung nach Anspruch 1, wobei die Bestrahlungsrichtung auf dem Monitor angezeigt wird.

3. Ultraschallbehandlungsvorrichtung nach Anspruch 2, wobei eine Anzeigeeinrichtung (40) für die Bestrahlungsrichtung anzeigt, daß die nach oben gerichtete Bewegung ausgewählt ist, indem das tomographische Bild auf dem Monitor kopfstehend dargestellt wird.

4. Ultraschallbehandlungsvorrichtung nach Anspruch 2, die außerdem eine Einrichtung zum Erzeugen graphischer Markierungen enthält, um zwei graphische Markierungen zu erzeugen, die jeweils den Tisch zum Tragen des Gegenstandes und einen Pfeil darstellen, der die Richtung des Applikators angibt.

5. Ultraschallbehandlungsvorrichtung nach einem der vorhergehenden Ansprüche, wobei die Applikatorhalteeinrichtung (20) ein auf einem Boden feststehendes Grundgestell (100), eine am Grundgestell (100) montierte Einrichtung (120) zum Verschieben in X-Y-Richtung, um den Applikator (10) in einer zweidimensionalen Ebene zu verlagern, eine am Grundgestell (100) angebrachte Stütze (110), eine an der Stütze (110) montierte Einrichtung (131, 132, 133 und 171) zum Verschieben in Z-Richtung, um den Applikator (10) senkrecht zur zweidimensionalen Ebene zu verlagern, einen Querträger (150) mit einer Drehachse (140), der entlang der Drehachse (140) durch die Stütze (110) gehalten wird, und eine zum Drehen des Querträgers (150) vorgesehene Dreheinrichtung (141, 142, 143 und 144) aufweist, um den Applikator (10) um die Achse (140) zu drehen, die entlang der zweidimensionalen Ebene horizontal angeordnet ist.

6. Ultraschallbehandlungsvorrichtung nach Anspruch 5, wobei die Erfassungseinrichtung (145) für die Bestrahlungsrichtung vier Sensoren (147a bis 147d) aufweist, die an der Stütze (110) angebracht sind, um den Drehwinkel der Drehachse (140) zu erfassen, indem ein Teil der sich drehenden Anordnung erfaßt wird, welches jeweils zwischen zwei Sensoren liegt.

7. Ultraschallbehandlungsvorrichtung nach Anspruch 6, wobei die Erfassungseinrichtung für die Bestrahlungsrichtung ein Drehpotentiometer (210) aufweist, um den Drehwinkel zu erfassen.

8. Ultraschallbehandlungsvorrichtung nach einem der Ansprüche 3 bis 7, wobei die Anzeigeeinrichtung (40) für die Bestrahlungsrichtung durch einen Umschalter (44) gesteuert werden kann, der von einem Bediener manuell betätigt wird.

9. Ultraschallbehandlungsvorrichtung nach Anspruch 8, die außerdem eine Schaltereinheit (43) aufweist, um die Anzeige der Bestrahlungsbedingung auszuwählen, die entweder automatisch durch die Erfassungseinrichtung (145) für die Bestrahlungsrichtung oder manuell durch den Umschalter (44) gesteuert wird.

## Revendications

1. Dispositif de thérapie par ultrasons ayant un applicateur (10) pour générer et appliquer un ultrason à un objet à traiter, et moyen de support d'applicateur (20) pour supporter l'applicateur (10) mobile dans différentes directions, ce dispositif comprenant:-
une sonde ultrasonique (18a) aménagée dans l'applicateur (10) pour acquérir des informations d'image tomographique de l'objet à examiner et à détruire; et
un moyen 144 pour changer la direction d'approche donnée à l'applicateur (10) qui peut être une approche ascendante ou une approche descendante par rapport à l'objet pour appliquer un traitement approprié de l'objet à traiter;
un moyen à moniteur (42) pour afficher les informations d'image tomographique de l'objet fournies par une sonde ultrasonique (18A);
caractérisé par le fait que cette sonde (18a) comprend un transducteur à deux couches (18-1 et 18-2) sur un élément servant de support (B) pour émettre de manière sélective des ultrasons à basse fréquence et à haute fréquence, un moyen de conversion de fréquence ultrasonore (23 et 24) comprenant un commutateur (23) prévu pour convertir en basse fréquence la fréquence ultrasonore émise par la sonde ultrasonique (18a), lorsqu'on actionne l'approche ascendante, ce moyen étant commuté automatiquement par ce commutateur (23) en réponse à la direction d'approche de la sonde ultrasonique (18a) détectée par le moyen de détection de direction d'approche (145) qui détecte si la direction d'approche est ascendante ou descendante.

2. Dispositif de thérapie par ultrasons selon la revendication 1 dans lequel la direction d'approche est indiquée sur le moniteur.

3. Dispositif de thérapie par ultrasons selon la revendication 2, dans lequel le moyen d'indiquer la direction d'approche (40) indique que le mouvement ascendant a été sélectionné par production d'une image tomographique renversée sur le moniteur.

4. Dispositif de thérapie par ultrasons selon la revendication 2, comprenant en outre un moyen de générer des points de repères graphiques pour générer deux marques graphiques qui représentent respectivement la table de support de l'objet et une flèche qui correspond à la direction donnée à l'applicateur.

5. Dispositif de thérapie aux ultrasons selon l'une quelconque des revendications qui précèdent dans lequel le moyen de support de l'applicateur (20) comporte une base (100) fixée au sol et un moyen de déplacement selon des coordonnées X-Y (120) monté sur la base (100) pour déplacer l'applicateur (10) sur un plan bi-dimensionnel, une tour (110) placée sur la base (100) un moyen de déplacement dans la direction 〈〈Z〉〉 (131, 132, 133 et 171) monté sur la tour (110) pour déplacer l'applicateur (10) perpendiculairement au plan bi-dimensionnel, et un élément en forme de bras (150) ayant un axe de rotation(140) supporté par la tour (110) le long de l'axe de rotation (140), et un moyen de rotation (141, 142, 143 et 144) prévu pour faire tourner l'élément en forme de bras (150) pour faire tourner l'applicateur (10) autour de l'axe (140) aménagé horizontalement le long du plan bi-dimensionnel.

6. Dispositif de thérapie par ultrasons selon la revendication 5, dans lequel le moyen de détection de la direction d'approche (145) comporte des capteurs (147a à 147d) branchés à la tour pour détecter l'angle de rotation de l'axe de rotation (140) en détectant une partie de la structure rotative qui s'étend entre les deux capteurs.

7. Dispositif de thérapie par ultrasons selon la revendication 6, dans lequel le moyen de détection de la direction d'approche comporte un potentiomètre rotatif (210) pour détecter l'angle de rotation.

8. Dispositif de thérapie par ultrasons selon l'une quelconque des revendications 3 à 7, dans lequel le moyen d'indication de la direction d'approche (40) peut être contrôlé à l'aide d'un commutateur rotatif (44) manoeuvré à la main par l'opérateur.

9. Dispositif de thérapie aux ultrasons selon la revendication 8, comprenant en outre une unité de commutation (43) pour sélectionner l'indication de condition d'approche soit par contrôle automatique d'après le moyen de détection de direction d'approche (145) soit par contrôle manuel à l'aide du commutateur rotatif (44).
